# EUROPÄISCHE PATENTSCHRIFT

(11) **EP 0 980 266 B1**
(45) Veröffentlichungstag und Bekanntmachung des Hinweises auf die Patenterteilung: **17.07.2002**
(21) Anmeldenummer: 98928215.7
(22) Anmeldetag: 21.04.1998
(51) Int. Cl.: A61L 2/00, A61L 11/00

(54) **VERFAHREN UND VORRICHTUNG ZUR BEHANDLUNG VON KONTAMINIERTEN, INSBESONDERE INFIZIERTEN MATERIALIEN**
METHOD AND APPARATUS FOR TREATING CONTAMINATED MATERIAL, SPECIFICALLY INFECTED MATERIAL
PROCEDE ET DISPOSITIF DE TRAITEMENT DE MATERIAUX CONTAMINES, NOTAMMENT INFECTES

(30) Priorität: 26.04.1997 DE 19717839
(43) Veröffentlichungstag der Anmeldung: 23.02.2000
(73) Patentinhaber: Göldner, Helmut, 31633 Leese (DE)
(72) Erfinder: Göldner, Helmut, 31633 Leese (DE)
(74) Vertreter: Braun, Dieter, Dipl.-Ing.
(86) Internationale Anmeldenummer: EP9802371
(87) Internationale Veröffentlichungsnummer: WO9848853

(56) Entgegenhaltungen:
- EP-A- 0 393 231
- EP-A- 0 672 426
- DE-A- 4 138 939
- DE-C- 3 938 546
- US-A- 3 464 342
- US-A- 3 617 313

## Beschreibung

Die Erfindung bezieht sich auf Verfahren und eine Vorrichtung zur Behandlung von kontaminierten, insbesondere infizierten Materialien, bei dem diese über einen Einwurftrichter einer Förderschnecke zugeführt, aufgeheizt und desinfiziert bzw. sterilisiert, sowie an einem Auswurf ausgetragen werden.

Durch die DE 39 38 546 C2 ist bereits eine Hochtemperatur-Desinfektionsanlage für krankenhausspezifische Abfälle bekannt geworden, bei der die Abfälle über einen Einlaßtrichter zwei durch eine druckdichte mechanische Zwischenschleuse getrennten Schneckenstreckenabschnitten zugeführt werden. Dabei wird im ersten Schneckenstreckenabschnitt durch Wärmeeinleitung ein Dampfdruck einstellbar erzeugt, während im zweiten Schneckenstreckenabschnitt ein Unterdruck erzeugt wird, um das Gut durch Absaugen der Dämpfe zu entfeuchten. Bei dieser bekannten Anlage ist es von Nachteil, daß mechanische Druckschleusen verwendet werden, die aufwendig und zudem störanfällig sind. Da stets mit Undichtigkeiten gerechnet werden muß, sind derartige mechanische Druckschleusen zur Sicherstellung der für eine Desinfektion erforderlichen Druckverhältnisse nicht geeignet Hinzu kommt, daß die Schneckenstreckenabschnitte in der horiziontalen Ebene angeordnet sind, so daß kontaminierte Flüssigkeit unbemerkt durch die Anlage fließen und diesen Prozeß unbehandelt bzw. unzulänglich behandelt passieren kann. Es ist hier keineswegs sichergestellt, daß unter allen Bedingungen eine sichere Desinfektion bzw. insbesondere auch Sterilisation der Abfälle erfolgt.

Aus der US-A 3464342 ist eine Kochvorrichtung zur Verarbeitung von Tierabfällen zu Viehfutter bekannt, bei der ein zu behandelndes Material mit einer ersten Förderschnecke an deren Ende zu einem Materialpfropf verdichtet wird und zwecks Wärmebehandlung in eine zweite unter Druck arbeitende Förderschnecke geleitet wird, an deren Ende über eine dritte Förderschnecke der weitere zur Druckbildung nötige Materialpfropf gebildet wird.

Der Erfindung liegt die Aufgabe zugrunde, ein Verfahren und eine Vorrichtung zu schaffen, die unter Einsatz einfacher technischer Mittel stets eine sichere Desinfektion bzw. auch Sterilisation kontaminierter Materialien sicherstellt und bei der zusätzlich der Energiebedarf optimiert ist.

Diese Aufgabe wird erfindungsgemäß durch die Merkmale des Anspruchs 1 sowie des Anspruchs 4 gelöst. Die weitere Ausgestaltung der Erfindung ist den Unteransprüchen zu entnehmen.

Dadurch, daß die Materialien einer ersten Förderschnecke zugeführt werden, die als Aufheizschnecke ausgebildet ist, in der Aufheizschnecke zusätzlich eine Verdichtung des Materials in der Form erfolgt, daß in deren Endbereich ein abdichtender Materialpfropfen erzeugt wird, am Ende der Aufheizschnecke das Material in eine Behandlungskammer freigegeben wird und sich hier entspannt, das Material im Bereich der Behandlungskammer in eine zweite Förderschnecke gelangt, die als Behandlungsschnecke ausgebildet ist, in die Behandlungskammer und in die Behandlungsschnecke Energie stoßweise definiert eingeleitet, Wasserdampf zugeführt oder erzeugt und ein Überdruck und die erforderliche Temperatur aufgebaut und gehalten wird und im Endbereich der Behandlungsschnecke das Material zu einem zweiten abdichtenden Materialpfropfen verdichtet wird, ist erreicht worden, daß zwischen den beiden sich ständig selbsttätig ausbildenden, gut abdichtenden Materialpfropfen der Schnecken über einen langen Weg ein für das Verfahren sehr vorteilhafter Überdruck für einen definierten Zeitraum gehalten werden kann.

Vorzugsweise wird das Material in der Aufheizschnecke, die in ihrer Förderrichtung schräg nach oben geneigt angeordnet ist, nach oben transportiert. Hierdurch wird sicher verhindert, daß kontaminierte Flüssigkeit aus dem Bereich unterhalb des Einwurftrichters unbemerkt in die Anlage fließen und den Prozeß unbehandelt bzw. unzulänglich behandelt passieren kann. Der Neigungswinkel der Aufheizschnecke zur horizontalen Ebene liegt in der Regel zwischen 20° und 45°.

Es kann in die Behandlungskammer und in die Behandlungsschnecke Sattdampf stoßweise definiert eingeleitet werden. Durch die stoßweise Einleitung wird eine Druckerhöhung und eine Tiefenwirkung erreicht, da zwischen den Dampfstößen eine Entspannung, insbesondere des in der Behandlungskammer befindlichen Materials, erfolgt. Ein gleicher Effekt ist erzielbar, sofern in der Behandlungskammer und/oder der Behandlungsschnecke Mikrowellenenergie in feuchtes Material stoßweise definiert eingeleitet wird.

Im Rahmen der Erfindung ist kann es vorgesehen werden, daß am Eingang der Aufheizschnecke mittels eines Feuchtesensors der Feuchtigkeitsgehalt des Materials gemessen und daß bei Bedarf dem Material Wasser in einer erforderlichen Dosierung zugesetzt wird.

Wenn am Ende der Aufheizschnecke die Temperatur des Materials gemessen und in Abhängigkeit von der gemessenen Temperatur der Antrieb der Aufheizschnecke derart gesteuert wird, kann die für eine bestimmte Temperatur vorgeschriebene Verweilzeit des Materials in den Förderschnecken und in der Behandlungskammer sichergestellt werden.

Weiterhin kann vorgesehen werden, daß im Bereich am Eingang der Behandlungsschnecke mittels eines Temperaturfühlers der Temperaturverlauf erfaßt und mittels eines Aufzeichnungsgerätes festgehalten und dokumentiert wird, so daß der Temperaturverlauf zu Kontrolizwecken nachträglich reproduzierbar ist. Es ist somit möglich, auch sehr viel später den Nachweis zu führen, daß das Material vorschriftsmäßig behandelt wurde. Sofern im hinteren Bereich der Behandlungsschnecke ein Temperaturfühler angeordnet ist, mit dem die Temperatur des Materials in diesem Bereich gemessen und mittels eines Aufzeichnungsgerätes aufgezeichnet wird, ist eine Auswertung der Daten im Vergleich mit den Aufzeichnungen des Temperaturfühlers im Eingabebereich der Behandlungsschnecke möglich.

Vorteilhaft kann es sein, wenn im Endbereich der Aufheizschnecke und der Behandlungsschnecke jeweils ein konusförmiger Ring angeordnet ist, der die Verdichtung des Materials und die Ausbildung der abdichtenden Pfropfen begünstigt. Hinter dem Ring erfolgt dann eine Entspannung des Materials.

Die Verdichtung des Materials kann im Bereich der Förderschnecke und/oder der Behandlungsschnecke mittels einer Transportspirale erfolgen. Hier ist eine besonders gute und sichere Ausbildung der abdichtenden Pfropfen erzielbar, da das Material auch aufgrund der fehlenden inneren Schneckenwelle besonders intensiv in dem Endbereich der Schnecken gefördert und dort intensiv verdichtet wird. Dieser Effekt kann gesteigert werden, indem die Verdichtung des Materials in der Förderschnecke und/oder der Behandlungsschnecke in der Form erfolgt, daß sich der Steigungswinkel der Transportspirale im Endbereich verringert.

Im Rahmen der Erfindung kann es außerdem vorgesehen werden, daß mittels einer am Ende der Behandlungsschnecke angeordneten Rückhalteklappe eine zusätzliche Verdichtung des Materials erfolgt. Sofern die zusätzliche Verdichtung mittels einer mehrteiligen Rückhalteklappe erfolgt, wird ein stets wirksamer zusätzlicher Drosselspalt aufrechterhalten.

Im Bereich des Auswurfs austretende Vrasen können mittels eines Absaugschachtes abgesaugt werden.

Indem in der Behandlungskammer oder im Einlauf der Behandlungsschnecke ein Temperaturfühler angeordnet ist, kann mittels dem die Behandlungstemperatur des Materials erfaßt und der Antrieb der Behandlungsschnecke zur Aufrechterhaltung der optimalen Behandlungstemperatur und Behandlungszeit geregelt werden.

Die erfindungsgemäße Vorrichtung ist im übrigen dadurch gekennzeichnet, daß eine erste Förderschnecke vorgesehen ist, die als von einem Heizelement umgebende Aufheizschnecke ausgebildet ist und in der eine Verdichtung des Materials in der Form erfolgt, daß in deren Endbereich ein abdichtender Materialpfropfen erzeugt wird, daß am Ende der ersten Förderschnecke eine Behandlungskammer angeordnet ist, in der eine Entspannung und Auflockerung der Struktur des Materials erfolgt, daß in die Behandlungskammer eine zweite Förderschnecke eingreift, die als von einem Heizelement umgebende Behandlungsschnecke für das Material ausgebildet ist, daß in die Behandlungskammer und in die Behandlungsschnecke Energie stoßweise definiert einleitbar ist, Wasserdampf zugeführt oder/und erzeugt und ein Überdruck und die erforderliche Temperatur aufgebaut und gehalten werden und daß die Behandlungsschnecke das Material in ihrem Endbereich zu einem zweiten abdichtenden Materialpfropfen verdichtet, derart, daß zwischen den beiden als Dichtung wirkenden Materialpfropfen der Schnecken der Überdruck für einen definierten Zeitraum gehalten werden kann.

Im Rahmen der Erfindung ist es vorgesehen, daß die Aufheizschnecke und/oder die Behandlungsschnecke von einer Transportspirale gebildet ist, die auf sich entlang der Innenwandungen der Schneckengehäuse in deren Längsrichtung erstreckenden Verschleißschienen gelagert ist. Dabei kann es vorteilhaft sein, wenn sich der Steigungswinkel der Aufheizschnecke und/oder der Behandlungsschnecke in deren Endbereich verringert.

Gemäß einer bevorzugten Ausführungsform der Erfindung ist die Aufheizschnecke in der Vorrichtung in Förderrichtung schräg nach oben geführt angeordnet. Es wird so verhindert, daß unzulässig große Mengen Flüssigkeit in die Behandlungskammer bzw. in die Behandlungsschnecke gefördert werden. Die Aufheizschnecke kann in einem Winkel von 20° bis 45° schräg nach oben geführt werden.

In der Zeichnung ist ein Ausführungsbeispiel der Erfindung dargestellt und wird im folgenden näher beschrieben. Es zeigen:

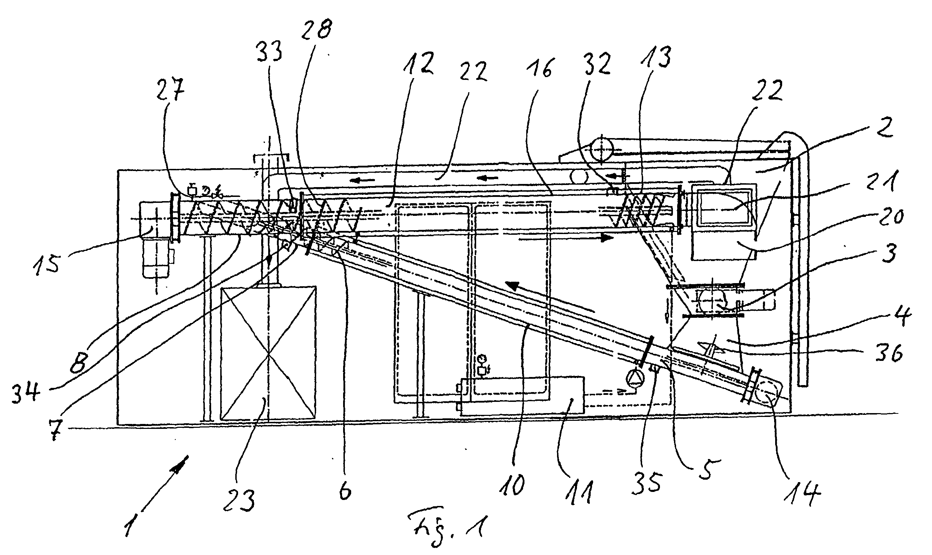

Figur 1, eine schematische Seitenansicht einer Vorrichtung;

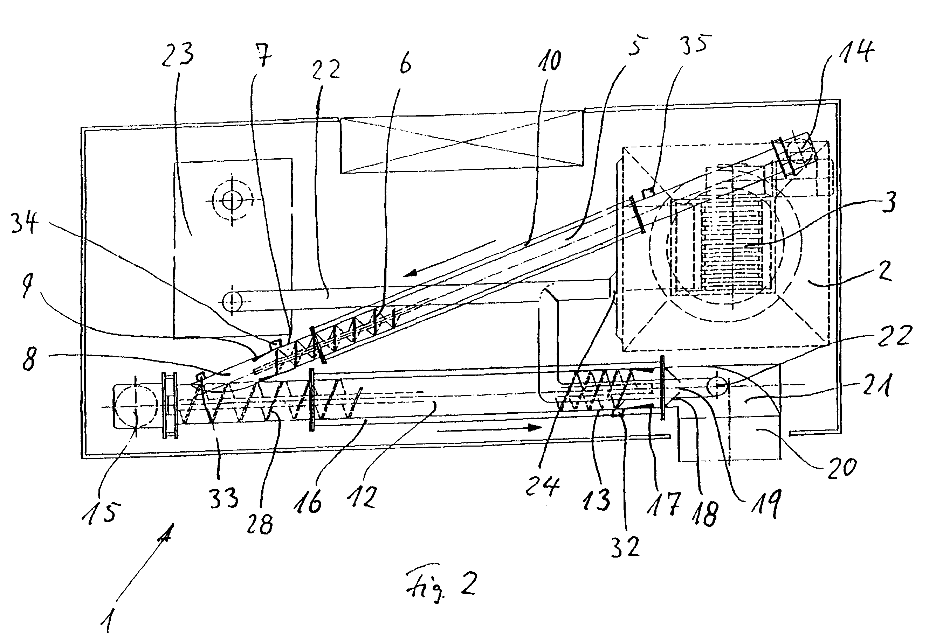

Figur 2, die Vorrichtung gemäß Figur 1 in einer Draufsicht;
Figur 3, die Aufheizschnecke im Querschnitt.

In der Zeichnung ist mit 1 eine Vorrichtung zur Behandlung von kontaminierten, insbesondere infizierten Materialien bezeichnet. Die Vorrichtung 1 weist einen Einwurftrichter 2 auf, unter dem ein Zerkleinerer 3 angeordnet ist. Von dem Zerkleinerer 3 führt ein Schacht 4 an eine erste Förderschnecke, die als Aufheizschnecke 5 ausgebildet ist. Die Aufheizschnecke 5 ist in einem Neigungswinkel zur horizontalen Ebene, der bei ca. 30° liegt, angeordnet. Die Aufheizschnecke 5 weist eine Transportspirale 6 auf, die einen Endbereich 7 aufweist, der in eine Behandlungskammer 8 einmündet. Im Endbereich 7 der Aufheizschnecke 5 ist ein konusförmiger Ring 9 angeordnet.

Die Aufheizschnecke 5 ist über den größten Teil ihrer Länge mit einem Doppelmantel 1 0 versehen, in dem sich Wärmeträgeröl befindet, das in einem Aufheizblock 11 erwärmt wird. Die Aufheizschnecke 5 ist im übrigen in Förderrichtung schräg nach oben in die Behandlungskammer 8 der Vorrichtung 1 geführt. Der Neigungswinkel der Aufheizschnecke zur horizontalen Ebene liegt vorteilhafterweise zwischen 20° und 45°. Die Transportspirale 6 der Aufheizschnecke 5 weist im übrigen in ihrem Endbereich 7 einen verringerten Steigungswinkel auf.

Die Behandlungskammer 8 der Vorrichtung 1 ist an eine zweite Förderschnecke geführt, die als Behandlungsschnecke 12 ausgebildet ist. Die Behandlungsschnecke 12 weist eine Transportspirale 28 auf, die in einem Endbereich 13 der Behandlungsschnecke 12 eine verringerte Steigung aufweist. Die Aufheizschnecke 5 und die Behandlungsschnecke 12 sind über Antriebe 14 und 15 angetrieben.

Auch die Behandlungsschnecke 12 ist mit einem Doppelmantel 16 versehen, der sich über den größten Teil ihrer Länge erstreckt und mit Wärmeträgeröl gefüllt ist. Auch dieses Wärmeträgeröl wird in dem Aufheizblock 11 aufgeheizt. Innerhalb der Behandlungsschnecke 12 ist in deren Endbereich 13 ein konusförmiger Ring 17 angeordnet. Der Endbereich 13 ist durch eine Rückhalteklappe 18 abgeschlossen, die hier einen Drosselspalt 19 bildet. An die Rückhalteklappe 18 der Behandlungsschnecke 12 schließt sich ein Auswurf 21 mit einer Rutsche 20 an. Im Bereich des Auswurfs 21 ist ein Absaugschacht 22 angeordnet, der an eine Filteranlage 23 geführt ist. Ein weiterer Anschlußstutzen 24 des Absaugschachtes 22 ist an den Einwurftrichter 2 geführt.

Im Endbereich 7 der Aufheizschnecke 5 bildet sich bei Betrieb der Vorrichtung 1 ein abdichtender Materialpfropfen von ca. 100 bis 200 mm Länge aus. Auch im Endbereich 13 der Behandlungsschnecke 12 bildet sich ein abdichtender Materialpfropfen aus. Im Bereich der Behandlungskammer 8 ist ein Einlaß 27 für Sattdampf angeordnet.

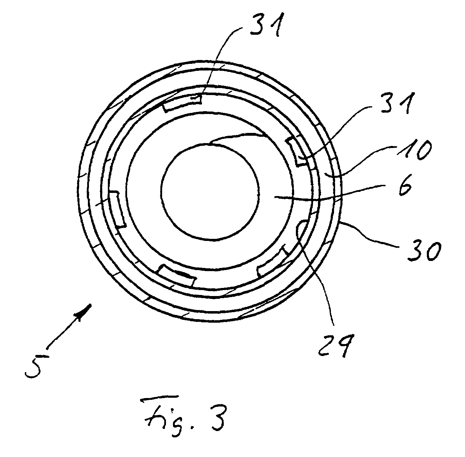

Die Transportspiralen 6 und 28 der Aufheizschnecke 5 und der Behandlungsschnecke 12 sind, wie aus der Figur 3 ersichtlich ist, auf sich entlang der Innenwandungen 29 der Schneckengehäuse 30 in deren Längsrichtung erstreckenden Verschleißschienen 31 gelagert. Die Verschleißschienen 31 bewirken als Zusatzeffekt eine vorteilhafte Scherung und Zwangsföhrung des transportierten Materials.

Im hinteren Bereich der Behandlungsschnecke 12 ist ein Temperaturfühler 32 angeordnet. Ein weiterer Temperaturfühler 33 ist im Bereich der Behandlungskammer 8 angeordnet. Zusätzlich ist ein Temperaturfühler 34 am Ende der Aufheizschnecke 5 angeordnet. Ein Feuchtesensor 35 ist am Eingang der Aufheizschnecke 5 angeordnet.

Zur Behandlung von kontaminierten Materialien wird der Vorrichtung 1 über den Einwurftrichter 2 das Material zugeführt. Der Zerkleinerer 3 zerkleinert das Material auf eine Größe von vorzugsweise 10 x 20 mm, wobei Hohlkörper, wie Spritzen, zerstört werden. Das Material wird dann über den Schacht 4 der Aufheizschnecke 5 zugeführt. Der zwischengeschaltete Dosierer 36 sorgt dabei für einen optimalen Füllstand in der Aufheizspirale.

In der Aufheizsschnecke 5 wird das Material transportiert, auf ca. 105 °C erwärmt und verpresst. Da die Aufheizschnecke 5 in einem Winkel von ca. 30° zur horizontalen Ebene angeordnet ist, ergibt sich eine Aufwärtsförderung des Materials. Hierdurch wird sehr sicher verhindert, daß kontaminierte Flüssigkeit aus dem Bereich unterhalb des Einwurftrichters 2 unbemerkt in die Anlage fließen und den Prozeß unbehandelt bzw. unzulänglich behandelt passieren kann.

Die wellenlose Transportspirale 6 fördert das Material über eine vorgegebene Transportlänge in die Behandlungskammer 8. Da die Aufheizschnecke 5 über das in ihrem Doppelmantel 10 befindliche Wärmeträgeröl aufgeheizt wird, wird auch das in der Aufheizschnecke 5 befindliche Material definiert aufgeheizt. Wesentlich ist, daß das Material im Endbereich 7 der Aufheizschnecke 5 zu einem Materialpfropfen verdichtet wird. Der in diesem Bereich angeordnete konusförmige Ring 9 unterstützt diese Verdichtung des Materials. Außerdem begünstigt die abnehmende Steigung der Transportspirale 6 im Endbereich 7 die Verdichtung des Materials.

Hinter dem konusförmigen Ring der Aufheizschnecke 5 kommt es zu einer Auflockerung des Materials, das in die Behandlungskammer 8 gelangt. In die Behandlungskammer 8 wird stoßweise Sattdampf zugeführt und ein Überdruck und die erforderliche Temperatur aufgebaut und gehalten. Als Dampfphasen sind vorzugsweise ca. 2 Minuten Bedampfung und jeweils eine Minute Pause vorgesehen. Insbesondere werden durch die stoßweise Beaufschlagung auch im Material befindliche verschlossene Hohlkörper aufgebrochen.

Das Halten des Druckes des in der Behandlungskammer 8 befindlichen Sattdampfes ist sicher möglich, da sich auch im Endbereich 13 der sich anschließenden Behandlungsschnecke 12 der abdichtende Materialpfropfen ausbildet. Es können somit in der Behandlungskammer 8 sowie auch in der Behandlungsschnecke 12 optimale Bedingungen für eine Desinfektion und insbesondere auch Sterilisation des Materials eingestellt werden. Insgesamt ergibt sich eine sehr günstige Energiebilanz. Der stoßförmig eingeleitete Sattdampf bewirkt im übrigen eine Komprimierung und vorteilhafte Auflockerung des Materials in der Behandlungskammer 8, so daß der Dampf das Material sehr gut durchdringen kann. Hierdurch wird der Sterilisierungsprozeß optimiert. Der Sattdampf wird im übrigen in die Behandfungskammer 8 am Einlaß 27 zugeführt.

Das Material wird über die Behandlungsschnecke 12 in Richtung auf den Auswurf 21 transportiert. Dabei verdichtet die Transportspirale 28, deren Steigung im Endbereich 13 der Behandlungsschnecke 12 abnimmt, das Material zu einem abdichtenden Materialpfropfen. Auch hier unterstützt der konusförmige Ring 17 die Verdichtung des Materials. Der Drosselspalt 19 der Rückhalteklappe 18 unterstützt die Verdichtung zusätzlich. Das behandelte Material gelangt schließlich an den Auswurf 21 und wird über die Rutsche 20 abgeführt. Über den Absaugschacht 20 werden die sich bildenden Vrasen abgesaugt, wobei die abgesaugte Luft in der Filteranlage 23 gereinigt wird. Das gleiche gilt für die über den Absaugstutzen 24 aus dem Einwurftrichter 2 abgesaugte schadstoffbelastete Luft. Im übrigen erfolgt auch in der Behandlungsschnecke 12 eine zusätzliche Aufheizung des Materials über das im Doppelmantel 16 befindliche Wärmeträgeröl.

Sofern in die Behandlungskammer 8 bzw. in die Behandlungsschnecke 12 alternativ Mikrowellenenergie stoßweise eingeleitet werden soll, so kann über den Feuchtesensor 35, der am Eingang der Aufheizschnecke 5 angeordnet ist, der Feuchtigkeitsgehalt des Materials gemessen und bei Bedarf dem Material Wasser in einer erforderlichen Dosierung zugesetzt werden. Dieses Wasser stellt dann bei der nachfolgenden Energieeinleitung die Bildung einer ausreichenden Menge von Wasserdampf sicher.

Über einen im Bereich der Behandlungskammer 8 bzw. am Eingang der Behandlungsschnecke 12 angeordneten Temperaturfühler 33 kann der Temperaturverlauf während der Behandlung des Materials erfaßt und mittels eines in der Zeichnung nicht dargestellten Aufzeichnungsgerätes festgehalten und dokumentiert werden. Es ist somit jederzeit eine Kontrolle des Temperaturverlaufes auch sehr viel später möglich.

Mittels des im Endbereich 7 der Behandlungsschnecke 8 angeordneten Temperaturfühlers 32 ist es möglich, die Temperatur des Materials auch in diesem Bereich zu messen und mittels eines in der Zeichnung nicht dargestellten Aufzeichnungsgerätes aufzuzeichnen. Es ist so beispielsweise eine Auswertung der Daten im Vergleich mit den Aufzeichnungen des Temperaturfühlers 33 im Eingabebereich der Behandlungsschnecke 12 möglich. Insbesondere ist es auch möglich, die Antriebe 14 und 15 der Aufheizschnecke 5 und der Behandlungsschnecke 12 so zu steuern, daß sich optimale Behandlungstemperaturen und Behandlungszeiten ergeben.

Insgesamt zeichnet sich die erfindungsgemäße Vorrichtung dadurch aus, daß es sich um eine sehr wirtschaftliche Durchlaufsterilisation handelt und daß aufgrund der beiden abdichtenden Materialpfropfen, die während der Behandlung des Materials in der Aufheizschnecke 5 und in der Behandlungsschnecke 12 zwangsläufig ausgebildet werden, eine optimale Betriebssicherheit gegeben ist. Das bedeutet, daß die kontaminierten Marterialien, bei denen es sich vorzugsweise um krankenhausspezifische Abfälle, aber z. B. auch um Klärschlamm, kontaminierte Böden sowie Lebensmittel, wie Getreide und Gewürze, handeln kann, sicher desinfiziert und wahlweise sogar sterilisiert werden können. Durch die Anordnung eines Shredders und die stoßweise Dampfbeaufschlagung werden auch im Material befindliche Hohlkörper mit Flüssigkeiten aufgebrochen. Insbesondere auch im Vergleich zu einem Autoklaven ergibt sich zudem eine sehr vorteilhafte Energiebilanz, die zusätzlich verbessert wird, da die Abfälle bereits in der Aufheizschnecke 5 vorgeheizt werden und die anschließende Behandlung über einen vergleichsweise sehr langen Weg und Zeitraum im Bereich zwischen den beiden das System abdichtenden Materialpfropfen bei erhöhtem Druck mittels Sattdampf erfolgt.

## Patentansprüche

1. Verfahren zur Behandlung von kontaminierten, insbesondere von infizierten Materialien, bei dem diese über einen Einwurftrichter (2) Förderschnecken (5, 12) zugeführt, aufgeheizt und desinfiziert bzw. sterilisiert, sowie an einem Auswurf (21) ausgetragen werden, **dadurch gekennzeichnet, daß**
- die Materialien einer ersten Förderschnecke zugeführt werden, die als Aufheizschnecke ausgebildet ist,
- in der Aufheizschnecke (5) zusätzlich eine Verdichtung des Materials in der Form erfolgt, daß in deren Endbereich ein abdichtender Materialpfropfen erzeugt wird,
- am Ende der Aufheizschnecke (5) das Material in eine Behandlungskammer (8) freigegeben wird und sich hier entspannt,
- das Material im Bereich der Behandlungskammer (8) in eine zweite Förderschnecke gelangt, die als Behandlungsschnecke (12) ausgebildet ist,
- in die Behandlungskammer (8) und in die Behandlungsschnecke (12) Energie stoßweise definiert eingeleitet, Wasserdampf zugeführt oder erzeugt und ein Überdruck und die erforderliche Temperatur aufgebaut und gehalten werden,
- im Endbereich der Behandlungsschnecke (12) das Material zu einem zweiten abdichtenden Materialpfropfen verdichtet wird, derart, daß zwischen den beiden abdichtenden Materialpfropfen der Schnecken (5, 12) der Überdruck für einen definierten Zeitraum gehalten werden kann.

2. Verfahren nach Anspruch 1, **dadurch gekennzeichnet, daß** das Material in der Aufheizschnecke (5), die in ihrer Förderrichtung schräg nach oben geneigt angeordnet ist, nach oben transportiert wird.

3. Verfahren nach Anspruch 1 und 2, **dadurch gekennzeichnet, daß** im Endbereich der Aufheizschnecke (5) und der Behandlungsschnecke (12) jeweils ein konusförmiger Ring (9, 17) angeordnet ist, der die Verdichtung des Materials und die Ausbildung der abdichtenden Materialpfropfen begünstigt.

4. Vorrichtung (1) zur Behandlung von kontaminierten, insbesondere infizierten Materialien, bei dem diese über einen Einwurftrichter (2) einer Förderschnecke (5, 12) zugeführt, dort aufgeheizt und desinfiziert bzw. sterilisiert, sowie an einem Auswurf (21) ausgetragen werden, **dadurch gekennzeichnet, daß**
- eine erste Förderschnecke vorgesehen ist, die als von einem Heizelement umgebende Aufheizschnecke (5) ausgebildet ist und in der eine Verdichtung des Materials in der Form erfolgt, daß in deren Endbereich ein abdichtender Materialpfropfen erzeugt wird,
- am Ende der Aufheizschnecke (5) eine Behandlungskammer (8) angeordnet ist, in der eine Entspannung und Auflockerung der Struktur des Materials erfolgt,
- daß in die Behandlungskammer (8) eine zweite Förderschnecke eingreift, die als von einem Heizelement umgebende Behandlungsschnecke (12) für das Material ausgebildet ist,
- in die Behandlungskammer (8) und in die Behandlungsschnecke (12) Energie stoßweise definiert einleitbar ist, Wasserdampf zugeführt oder/und erzeugt und ein Überdruck und die erforderliche Temperatur aufgebaut und gehalten werden,
- die Behandlungsschnecke (12) das Material in ihrem Endbereich (13) zu einem zweiten abdichtenden Materialpfropfen verdichtet, derart, daß zwischen den beiden als Dichtung wirkenden Materialpfropfen der Schnecken (5, 12) der Überdruck für einen definierten Zeitraum gehalten werden kann.

5. Vorrichtung (1) nach Anspruch 4, **dadurch gekennzeichnet, daß** die Aufheizschnecke (5) in Förderrichtung schräg nach oben geführt ist.

6. Vorrichtung (1) nach Anspruch 4, **dadurch gekennzeichnet, daß** in die Behandlungskammer (8) und in die Behandlungsschnecke (12) über einen Einlaß (27) Sattdampf stoßweise definiert einleitbar ist.

7. Vorrichtung (1) nach Anspruch 4 und Anspruch 5 oder 6, **dadurch gekennzeichnet, daß** sich der Steigungswinkel der Aufheizschnecke (5) und/oder der Behandlungsschnecke (12) in deren Endbereich (7, 13) verringert.

8. Vorrichtung (1) nach Anspruch 4 und einem oder mehreren der Ansprüche 5 bis 7, **dadurch gekennzeichnet, daß** das Heizelement der Aufheizschnecke (5) als mit einem Wärmeträgeröl gefüllter Doppelmantel (10) ausgebildet ist.

9. Vorrichtung (1) nach Anspruch 4 und einem oder mehreren der Ansprüche 5 bis 8, **dadurch gekennzeichnet, daß** im Endbereich der Aufheizschnecke (5) und der Behandlungsschnecke (12) jeweils ein konusförmiger Ring (9, 17) angeordnet ist, derart, daß die Verdichtung des Materials begünstigt wird.

10. Vorrichtung (1) nach Anspruch 4 und einem oder mehreren der Ansprüche 5 bis 9, **dadurch gekennzeichnet, daß** in der Behandlungskammer (8) oder im Einlauf der Behandlungsschnecke (12) ein Temperaturfühler (33) angeordnet ist, derart, daß die Behandlungstemperatur des Materials erfaßt und der Antrieb (15) der Behandlungsschnecke (12) zur Aufrechterhaltung der optimalen Behandlungstemperatur und Behandlungszeit regelbar ist.

## Claims

1. A method of treating contaminated, especially infected materials, in which these are taken to conveyor helices (5, 12) via a throw-in funnel (2), heated and disinfected or sterilised, and extracted at a discharge (21), **characterised in that**
- the materials are taken to a first helical conveyor, which is constructed as a heating helix,
- in the heating helix (5) in addition a compression of the material occurs such that in its final region a compressed wad of material arises, which is created in its final region,
- at the end of the heating helix (5) the material is released into a treatment chamber (8) and relaxes here,
- the material in the treatment chamber (8) reaches a second conveyor helix, which is constructed as a treatment helix (12),
- into the treatment chamber (8) and the treatment helix (12) energy is introduced in defined pulses, water vapour is added or created and a pressure and the required temperature is built up and maintained,
- in the final region of the treatment helix (12) the material is compressed into a second packed wad of material, such that the pressure between the two packed wads of material in the helices (5, 12) can be maintained for a defined period.

2. A method according to Claim 1, **characterised in that** the material in the heating helix (5), which is arranged to be inclined obliquely upwards in the direction of conveying, is transported upwards.

3. A method according to Claim 1 and Claim 2, **characterised in that** a cone shaped ring (9, 17) is arranged in each case in the final region of the heating helix (5) and the treatment helix (12), which favours the compression of the material and the build up of the packed wads of material.

4. An arrangement (1) for the handling of contaminated, especially infected materials, in which they are taken to conveyor helices (5, 12) via a throw-in funnel (2), heated and disinfected or sterilised, and extracted at a discharge (21), **characterised in that**
- a first conveyor helix (5) is provided which is configured as a heating helix surrounded by a heating element and in which a compression of the material occurs such that in its final region a compressed wad of material is created,
- at the end of the heating helix (5) a treatment chamber (8) is arranged, in which occurs a relaxation and dispersal of the structure of the material,
- a second conveyor helix engages in the treatment chamber (8), which is surrounded by a heating element and configured as a treatment helix (12) for the material,
- in the treatment chamber (8) and in the treatment helix (12) energy can be introduced in defined pulses, water vapour introduced or/and created and pressure and the required temperature are built up and maintained,
- the treatment helix (12) compresses the material to a second packed wad of material in its final region (13) such that the pressure between the two packed wads of material in the helices (5, 12) can be maintained for a defined period.

5. An arrangement (1) according to Claim 4, **characterised in that** the heating helix (5) is inclined obliquely upwards in the direction of conveying.

6. An arrangement (1) according to Claim 4, **characterised in that** saturated steam can be introduced in defined pulses into the handling chamber (8) and into the treatment helix (12) via an inlet (27).

7. An arrangement (1) according to Claim 4 and Claim 5 or Claim 6, **characterised in that** the angle of rise of the heating helix (5) and/or the treatment helix (12) decreases in their end regions (7, 13).

8. An arrangement (1) according to Claim 4 and one or more of the Claims 5 to 7, **characterised in that** the heating element of the heating helix (5) is configured as a double skin (10) filled with a heat bearing oil.

9. An arrangement (1) according to Claim 4 and one or more of the Claims 5 to 9, **characterised in that** a cone shaped ring (9, 17) is arranged in each case in the final region of the heating helix (5) and the treatment helix (12), such that the compression of the material is improved.

10. An arrangement (1) according to Claim 4 and one or more of the Claims 5 to 9, **characterised in that** a temperature sensor (33) is arranged in the treatment chamber (8) or in the inlet to the treatment helix (12) such that the processing temperature of the material is captured and the drive (15) of the treatment helix (12) can be regulated to maintain the optimum processing temperature and processing time.

## Revendications

1. Procédé de traitement de substances contaminées, notamment infectées, dans lequel celles-ci sont amenées à des vis transporteuses (5, 12) par l'intermédiaire d'une trémie d'alimentation (2), chauffées et désinfectées ou stérilisées, ainsi qu'évacuées au niveau d'une sortie (21), **caractérisé en ce que**
- les substances sont amenées à une première vis transporteuse, qui est agencée sous la forme d'une vis chauffante,
- la substance est en outre comprimée dans la vis chauffante (5) de telle sorte qu'un tampon de substance assurant une étanchéité soit engendré dans la zone d'extrémité de cette dernière,
- au niveau de l'extrémité de la vis chauffante (5), la substance est libérée dans une chambre de traitement (8), où elle se détend,
- dans la zone de la chambre de traitement (8), la substance arrive dans une deuxième vis transporteuse qui est agencée sous la forme d'une vis de traitement (12),
- dans la chambre de traitement (8) et dans la vis de traitement (12), de l'énergie est introduite de façon intermittente et définie, de la vapeur d'eau est amenée ou engendrée, et une surpression et la température nécessaire étant engendrées et maintenues,
- dans la zone d'extrémité de la vis de traitement (12), la substance est comprimée en un deuxième tampon de substance assurant une étanchéité, de telle sorte que la surpression puisse être maintenue durant un intervalle de temps défini entre les deux tampons de substance assurant une étanchéité des vis (5, 12).

2. Procédé selon la revendication 1, **caractérisé en ce que** la substance est transportée vers le haut dans la vis chauffante (5) qui, dans son sens de transport, est disposée en étant inclinée obliquement vers le haut.

3. Procédé selon la revendication 1 ou 2, **caractérisé en ce qu'**une bague conique (9, 17) est disposée dans la zone d'extrémité de la vis chauffante (5) et de la vis de traitement (12), laquelle favorise la compression de la substance et la formation du tampon de substance assurant une étanchéité.

4. Dispositif (1) destiné au traitement de substances contaminées, notamment infectées, dans lequel celles-ci sont amenées par l'intermédiaire d'une trémie d'alimentation (2) à une vis transporteuse (5, 12), chauffées et désinfectées ou stérilisées dans cette dernière, ainsi qu'évacuées au niveau d'une sortie (21), **caractérisé en ce que**
- il est prévu une première vis transporteuse, qui est agencée sous la forme d'une vis chauffante (5) entourée d'un élément de chauffage, et dans laquelle la substance est comprimée de telle sorte qu'un tampon de substance assurant une étanchéité soit engendré dans la zone d'extrémité de celle-ci,
- une chambre de traitement (8) est disposée au niveau de l'extrémité de la vis chauffante (5), dans laquelle la structure de la substance est détendue et ameublie,
- une deuxième vis transporteuse débouche dans la chambre de traitement (8), laquelle est agencée sous la forme d'une vis de traitement (12) entourée d'un élément de chauffage pour la substance,
- dans la chambre de traitement (8) et dans la vis de traitement (12), de l'énergie peut être introduite de façon intermittente et définie, de la vapeur d'eau est amenée et/ou engendrée, et une surpression et la température nécessaire étant engendrées et maintenues,
- la vis de traitement (12) comprime dans sa zone d'extrémité (13) la substance en un deuxième tampon de substance assurant une étanchéité, de telle sorte que la surpression puisse être maintenue durant un intervalle de temps défini entre les deux tampons de substance faisant office d'étanchéité des vis (5, 12).

5. Dispositif (1) selon la revendication 4, **caractérisé en ce que**, dans le sens du transport, la vis chauffante (5) conduit obliquement vers le haut.

6. Dispositif (1) selon la revendication 4, **caractérisé en ce que** de la vapeur saturée peut être introduite de façon intermittente et définie dans la chambre de traitement (8) et dans la vis de traitement (12) par l'intermédiaire d'une admission (27).

7. Dispositif (1) selon la revendication 4 et la revendication 5 ou 6, **caractérisé en ce que** l'angle d'inclinaison de la vis chauffante (5) et/ou de la vis de traitement (12) se réduit dans les zones d'extrémité (7, 13) de ces dernières.

8. Dispositif (1) selon la revendication 4 et l'une ou plusieurs des revendications 5 à 7, **caractérisé en ce que** l'élément de chauffage de la vis chauffante (5) est réalisé en tant que double enveloppe (10) remplie d'une huile caloporteuse.

9. Dispositif (1) selon la revendication 4 et l'une ou plusieurs des revendications 5 à 8, **caractérisé en ce qu'**une bague conique (9, 17) est disposée dans la zone d'extrémité de la vis chauffante (5) et de la vis de traitement (12), de telle sorte que la compression de la substance soit favorisée.

10. Dispositif (1) selon la revendication 4 et l'une ou plusieurs des revendications 5 à 9, **caractérisé en ce qu'**une sonde de température (33) est disposée dans la chambre de traitement (8) ou à l'entrée de la vis de traitement (12), de telle sorte que la température de traitement de la substance puisse être détectée, et que l'entraînement (15) de la vis de traitement (12) puisse être réglé pour le maintien optimal de la température de traitement et du temps de traitement.
